(19) Europäisches Patentamt
European Patent Office
Office européen des brevets

(11) Publication number: **0 018 682**
**A1**

# (12) EUROPEAN PATENT APPLICATION

(21) Application number: 80200353.3

(22) Date of filing: 17.04.80

(51) Int. Cl.³: C 07 D 303/48
A 61 K 31/335
//C07C121/76

(30) Priority: 04.05.79 US 36116

(43) Date of publication of application:
12.11.80 Bulletin 80/23

(84) Designated Contracting States:
BE DE FR GB NL

(71) Applicant: SHELL INTERNATIONALE RESEARCH MAATSCHAPPIJ B.V.
Carel van Bylandtlaan 30
NL-2596 HR DEN HAAG(NL)

(72) Inventor: Durham, Harry Graham
630 Elm Street
Modesto, California 95351(US)

(74) Representative: Keuzenkamp, Abraham et al,
c/o Shell Internationale Research Maatschappij B.V. P.O. Box 302
NL-2501 CH 's-Gravenhage(NL)

## (54) Oxiranecarboxylic acid ester derivatives, their preparation and their inclusion in lipogenesis inhibiting compositions.

(57) This invention relates to oxiranecarboxylic acid ester derivatives.

There are provided 3-substituted-2-(aminocarbonyl) oxiranecarboxylic acid esters of formula

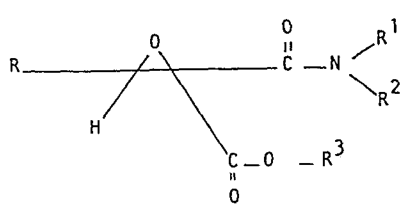

(I)

wherein R is phenyl substituted by one or two groups independently selected from nitro and alkoxy or is benzoyl, $R^3$ is alkyl, alkenyl, alkynyl or cycloalkyl, $R^1$ and $R^2$ are each independently hydrogen, alkyl, alkenyl, alkynyl or cycloalkyl or $R^1$ and $R^2$ together represent an alkylene chain containing four or five carbon atoms; processes for their preparation; lipogenesis-inhibiting compositions containing them and the preparation of such compositions.

Oxirane carboxylic acid ester derivatives of formula I have exhibited lipogenesis inhibiting activity.

EP 0 018 682 A1

Oxiranecarboxylic acid ester derivatives, their preparation and their inclusion in lipogenesis inhibiting compositions

This invention relates to oxiranecarboxylic acid ester derivatives, their preparation and their use as lipogenesis inhibitors in mammals.

Certain 3-substituted-2-(aminocarbonyl)oxiranecarboxylic acid ester derivatives, particularly ethyl 2-(aminocarbonyl)-3-phenyl-oxiranecarboxylate, are described, together with their preparation, by A. Robert and A. Foucaud in Bull. Soc. Chim. France, 1969, 2537-44. Lipogenesis-inhibiting properties of certain such ester derivatives are disclosed in Belgian Patent No. 865,625.

The Applicants have now discovered a novel class of 3-substituted-2-(aminocarbonyl)oxiranecarboxylic acid esters having lipogenesis activity in mammals.

According to the invention there is provided a 3-substituted-2-(aminocarbonyl)oxiranecarboxylic acid ester of formula

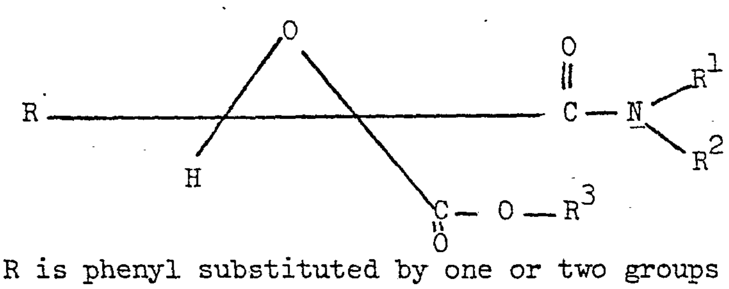

wherein R is phenyl substituted by one or two groups independently selected from nitro and alkoxy or is benzoyl, $R^3$ is alkyl, alkenyl, alkynyl or cycloalkyl, $R^1$ and $R^2$ are each independently hydrogen, alkyl, alkenyl, alkynyl or cycloalkyl or $R^1$ and $R^2$ together represent an alkylene chain containing four or five carbon atoms.

Although each alkyl, alkenyl or alkynyl moiety may be of straight-chain or branched-chain configuration, and of up to twenty

carbon atoms, and a cycloalkyl moiety may contain from three to ten carbon atoms, preferably the or each alkyl, alkenyl, alkynyl or cycloalkyl moiety in an ester of formula I contains up to 6 carbon atoms.

In preferred esters of formula I, $R^1$ and $R^2$ are both hydrogen atoms and $R^3$ is alkyl of from 1 to 4 carbon atoms.

Compounds of the invention can exist as either of two geometrical (cis-trans) isomers, depending upon the spatial relationship of the moieties upon the oxirane ring. Further, chirality exists in the compounds due to the asymmetric configurations at the 2-and 3-positions of the oxirane ring. As a result, four optical isomers exist, one pair for each of the two geometrical isomers. Both the cis and trans isomer products, as prepared, have been found to inhibit lipogenesis. The individual optical isomers have not been separated, so that their respective activity as lipogenesis inhibitors has not been determined. In this specification, for the sake of simplicity, these compounds are referred to generally as esters of 3-substituted-2-(aminocarbonyl)-oxiranecarboxylic acids, this terminology including each of the isomers, as well as mixtures thereof. Under the circumstances, the invention contemplates each of the individual isomers, as well as mixtures thereof.

The esters of formula I are conveniently prepared in accordance with the invention by epoxidising a compound of formula II

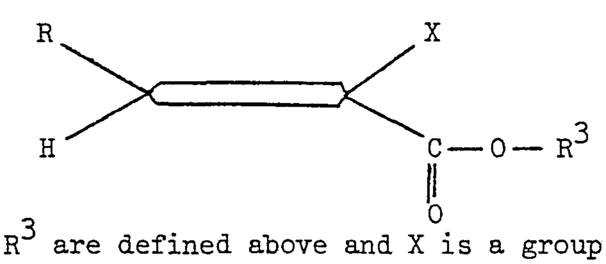

(II)

where R and $R^3$ are defined above and X is a group convertible to a group formula III

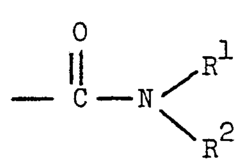

(III)

where $R^1$ and $R^2$ are as defined above, and converting X in to the group of formula III.

Epoxidation may be effected in known manner, e.g. using hydrogen peroxide, organic peracids or aqueous sodium or potassium hypochlorite solution.

When $R^1$ and $R^2$ in the eventual compound of formula I are both hydrogen atoms, X in the compound of formula II may conveniently be a cyano group, and the epoxidation and conversion of X may advantageously be effected in a single reaction step involving treatment of the compound of formula II with aqueous hydrogen peroxide solution in a polar solvent, e.g. a $C_{1-4}$ alkanol, in the presence of a base, e.g. sodium carbonate or tribasic sodium phosphate, conveniently at elevated temperature e.g. a temperature in the range from 40 to 70°C.

More generally, X may be a group of formula

$$-\overset{O}{\overset{\|}{C}}-O-R^3 \qquad (IV)$$

where $R^3$ is as defined above. Epoxidation may conveniently be carried out as described above and conversion of X into the group of formula III is then subsequently effected by reaction with an amine of formula $NHR^1R^2$ in a polar solvent, e.g. a $C_{1-4}$ alkanol at a moderate temperature, e.g. in the range from ambient temperature to 40°C, conveniently about 30°C.

Epoxidation conditions are also described in Bull. Soc. Chim. France, 1969, 2537-44.

When R in the is substituted phenyl, the intermediate of formula II is conveniently prepared by a Knoevenagel reaction between the appropriate benzaldhyde derivative and the appropriate X-substituted acetate.

When R is benzoyl, the intermediate of formula II is conveniently prepared by base-catalysed alkylation between a 2-haloacetophenone and the appropriate X-substituted acetate, followed by dehydrogenation of the resulting compound.

The esters of formula I can be used to control lipogenesis in warm-blooded animals such as, for example, pets, animals in a zoo,

livestock, fur-bearing animals and domestic animals, including, but not limited to dogs, cats, mink, goats, swine, cattle, horses, mules and donkeys. The effect is obtained by administering an effective amount of one or a mixture of two or more of the esters orally or parenterally to the animal. They may be administered as such, or as an active ingredient of a conventional pharmaceutical formulation. They may be administered orally by any covenient means. Thus, they may be orally administered as a drench, by intubation, in the animal's food and water, in a food supplement or in a formulation expressly designed for administration of the drug. Suitable formulations include solutions, suspensions, dispersions, emulsions, tablets, boluses, powders, granules, capsules, syrups and elixirs. For parenteral administration, they may be in the form of a solution, suspension, dispersion or emulsion. They can be administered in the form of an implant or other controlled sustained release formulation. Inert carriers, such as one or more of water, edible oil, gelatin, lactose, starch, magnesium stearate, talc or vegetable gum can be used. The dosage of the ester needed to inhibit lipogenesis will depend upon the particular ester used, and the particular animal being treated. However, in general, satisfactory results are obtained when the esters are administered in a dosage of from about 1 to about 400 milligrams per kilogram of the animal's body weight. The ester can be administered in a single dose or in a series of doses in the same day, or over a period of days. For any particular animal, a specific dosage regimen should be adjusted according to the individual need, the particular ester(s) used as the inhibitor, and the professional judgment of the person administering or supervising the administration of the inhibitor.

Accordingly the invention also provides a lipogenesis inhibiting composition comprising an ester of formula I in association with a pharmacentically or veterinarily acceptable carrier therefor. Further provided is a process for preparing a lipogenesis inhibiting composition which comprises bringing an ester of formula I into association with a pharmaceutically or veterinarily-acceptable carrier.

The invention also includes a method of inhibiting lipogenesis in a mammal which comprises administering to the mammal orally

or parentally an ester of formula I or a composition according to the invention.

The invention will be further understood from the following examples thereof, in which the identities of the products and the precursors therof were confirmed by appropriate and spectral analyses.

Example 1 Ethyl 2-(aminocarbonyl)-3-(3-nitrophenyl)oxiranecarboxylate (1)

A mixture of 134.5 g of 3-nitrobenzaldehyde, 101.7 g of ethyl cyanoacetate, 1 g of piperidine and 200 ml of acetic acid was heated on a steam bath for 1 hour. The acetic acid and unreacted 3-nitrobenzaldehyde were distilled off, to 100°C/0.02 Torr. The residue was extracted with hot hexane; no residue was left. The solution was chilled, and the solid that formed was recrystallized from methanol to give the ethyl ester of 2-cyano-3-(3-nitrophenyl)-2-propenoic acid (1A).

20 ml of 30% hydrogen peroxide was added over a 5-minute period to a mixture of 24.7 g of 1A, 100 ml of isopropyl alcohol and 1 ml of 1N sodium carbonate solution at 60°C. The mixture then was heated at 70°C for 1 hour and the solvent was evaporated under reduced pressure. 100 ml of water was added to the residue and the mixture was extracted with methylene chloride. The extract was dried (Celite) and the solvent was evaporated under reduced pressure. The residue was triturated with warm pentane, and recrystallized from isopropyl alcohol and dried in a vacuum oven to give (1), mp: 177-179°C.

Examples 2 to 4

By methods similar to that of Example 1 were prepared the following compounds:

(2) Ethyl 2-(aminocarbonyl)-3-(3,4-dimethoxyphenyl)oxirane carboxylate, mp: 177-178°C.

(3) Ethyl 2-(aminocarbonyl)-3-(4-methoxyphenyl)oxiranecarboxylate, mp: 140-141°C.

(4) Ethyl 2-(aminocarbonyl)-3-(4-nitrophenyl)oxiranecarboxylate, mp: 191-194°C.

Example 5 Ethyl 2-(aminocarbonyl)-3-benzoyloxiranecarboxylate (5)

10 g of sodium was dissolved in 200 ml of ethanol. The solution was cooled to 30°C and 45.2 g of ethyl cyanoacetate was added rapidly. The resulting mixture was cooled to -15°C and stirred while a warm solution of 62 g of 2-chloroacetophenone in 100 ml of toluene was added over a 15-minute period, with cooling, allowing the temperature of the mixture to rise to 20°C over a 1-hour period. The mixture was poured in ice/3N hydrochloric acid, the organic phase was separated, the solvent was evaporated under reduced pressure and the residue was distilled under reduced pressure to give a liquid, bp: 130-160°C, at 0.01 Torr., which solidified on standing. Recrystallization from cold ether gave the ethyl ester of 2-cyano-4-oxo-4-phenylbutanoic acid (5A), mp: 53-54°C.

A mixture of 11.5 g of 5A, 5.5 g of selenium dioxide and 30 ml of chlorobenzene was refluxed at 135°C. Then the chlorobenzene was distilled off under reduced pressure. The residue was extracted with hot hexane. The hexane was evaporated from the extract under reduced pressure. 30 ml of chlorobenzene was added to the residue and the mixture was refluxed overnight. The chlorobenzene was evaporated under reduced pressure. The residue was extracted with ether. The extract was cooled to give a solid, which was recystallized from hexane to give the E-isomer of the ethyl ester of 2-cyano-4-oxo-4-phenyl-2-butenoic acid (5B) mp: 74-75°C.

A mixture of 10 g of 5B, 100 ml of ethanol, 2 g of tribasic sodium phosphate (hydrate) and 15 ml of 30% hydrogen peroxide solution was heated at 60°C for 2 hours, then concentrated under reduced pressure. The residue was dissolved in methylene chloride. The solution was washed with water, concentrated to 25 ml. and filtered. The solid was recrystallized from acetone to give 5, mp: 148-149°C.

Compounds of formula I have been found to inhibit lipogenesis in tissues of mammals. The manner in which they cause this effect is not known with certainty; it is believed that they interfere with the synthesis of fatty acids in the tissues. Their effectiveness

for this purpose has been ascertained by immersing samples of swine adipose tissue in a liquid medium containing radioactive glucose and the test chemical, for a period of time, then isolating the lipid from the treated tissue and determining the incorporation of the radioactive carbon into lipid by means of scintillation counting techniques. These tests were conducted in swine adipose tissue because in swine, the primary site of lipogenesis -- i.e., fatty acid synthesis -- appears to be adipose tissue.

Described in more detail, the tests were conducted according to the following general procedure:

150 milligrams of slices of swine adipose tissue were incubated at 37°C for 2 hours with shaking in 3 milliliters of Krebs-Ringer bicarbonate solution containing one-half the normal calcium ion concentration, 60 micromoles of glucose, 0.5 micro-Curie of glucose-$U^{14}C$, and 300 microunits of insulin, and 5% dimethyl sulfoxide (DMSO). The test compounds were added as suspensions or solutions in DMSO and were present at a concentration of 100 micrograms per milliliter of the incubation mixture.

The incubation was terminated by addition of 0.25 milliliter of 1 N sulfuric acid. The resulting mixture was extracted with a total of 25 milliliters of chloroform: methanol (2:1 v/v). The extracts were washed according to Folch et al. (J. Biol. Chem., 226, 497-509, (1957)), air dried, and counted in a liquid scintillation counter with 15 milliliters of counting fluid (two parts toluene containing 0.4% w/v New England Nuclear Omifluor: 1 part Triton X-100). The tests in which all ingredients, proportions and conditions were the same except that no test compound was included. From the data obtained were calculated the percent inhibition of lipid synthesis by the test compound in each case. The data obtained from the tests are set out in Table 1, as the percent inhibition of lipogenesis compared to the results obtained in the control tests wherein only the test compound was omitted.

Table 1

| Compound No. | Percent Inhibition |
|---|---|
| 1 | 76 |
| 2 | 33 |
| 3 | 55 |
| 4 | 37 |
| 5 | 74 |

## CLAIMS

1. A 3-substituted-2-(aminocarbonyl)oxiranecarboxylic acid ester of formula

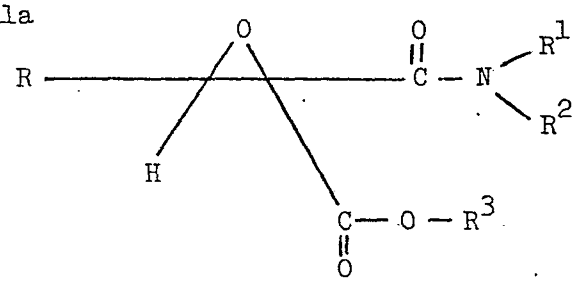

(I)

wherein R is phenyl substituted by one or two groups independently selected from nitro and alkoxy or in benzoyl, $R^3$ is alkyl, alkenyl, alkynyl or cycloalkyl, $R^1$ and $R^2$ are each independently hydrogen, alkyl, alkenyl, alkynyl or cycloalkyl or $R^1$ and $R^2$ together represent an alkylene chain containing four or five carbon atoms.

2. An ester according to Claim 1 further characterised in that the or each alkyl, alkenyl, alkynyl or cycloalkyl moiety contains up to 6 carbon atoms.

3. An ester according to Claim 1 or 2 further characterised in that $R^1$ and $R^2$ are both hydrogen atoms and $R^3$ is alkyl of from 1 to 4 carbon atoms.

4. A process for preparing an ester of formula I as defined in any one of Claims 1 to 3 which process is <u>characterised by</u> epoxidising a compound of formula II

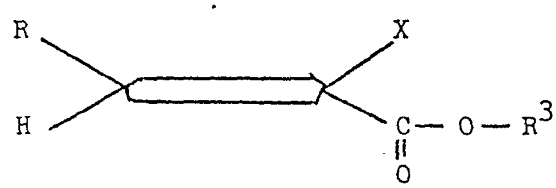

(II)

where R and $R^3$ are as defined in Claim 1 and X is a group convertible to a group of formula III

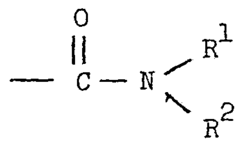

(III)

where $R^1$ and $R^2$ are as defined in Claim 1, and converting X into the group of formula III.

5. A lipogenesis inhibiting composition comprising an ester of formula I as defined in any one of Claims 1 to 3 in association with a pharmaceutically or veterinarily acceptable carrier therefor.

6. A process for preparing a lipogenesis inhibiting composition which comprises bringing an ester of formula I as defined in any one of Claims 1 to 3 into association with a pharmaceutically or veterinarily acceptable carrier.